(19) Europäisches Patentamt
European Patent Office
Office européen des brevets

(11) EP 1 611 386 B1

(12) **EUROPEAN PATENT SPECIFICATION**

(45) Date of publication and mention
of the grant of the patent:
**27.02.2013 Bulletin 2013/09**

(21) Application number: **04721592.6**

(22) Date of filing: **18.03.2004**

(51) Int Cl.:
*F16L 11/12* (2006.01)    *F16L 9/00* (2006.01)
*B29C 53/58* (2006.01)    *B01J 8/06* (2006.01)
*B29C 53/14* (2006.01)    *B29C 47/00* (2006.01)

(86) International application number:
**PCT/GB2004/001163**

(87) International publication number:
**WO 2004/083705 (30.09.2004 Gazette 2004/40)**

(54) **HELICAL PIPING**

SPIRALROHRLEITUNG

TUYAUX HELICOIDAUX

(84) Designated Contracting States:
**AT BE BG CH CY CZ DE DK EE ES FI FR GB GR HU IE IT LI LU MC NL PL PT RO SE SI SK TR**

(30) Priority: **18.03.2003 GB 0306179**

(43) Date of publication of application:
**04.01.2006 Bulletin 2006/01**

(60) Divisional application:
**09001557.9 / 2 090 267**

(73) Proprietor: **Technip France S.A.S.**
**92400 Courbevoie (FR)**

(72) Inventors:
• **CARO, Colin Gerald**
**London SW7 2AZ (GB)**
• **WATKINS, Nicholas V.**
**London SW7 2AZ (GB)**

(74) Representative: **Stevens, Jason Paul et al**
**Dehns**
**St Bride's House**
**10 Salisbury Square**
**London**
**EC4Y 8JD (GB)**

(56) References cited:
**EP-A- 0 712 711    GB-A- 499 058**
**GB-A- 2 192 966    US-A- 2 832 374**
**US-A- 3 610 289    US-A- 5 167 483**
**US-A- 5 711 744**

• **PATENT ABSTRACTS OF JAPAN vol. 006, no. 089 (M-132), 27 May 1982 (1982-05-27) & JP 57 027740 A (KINUGAWA RUBBER IND CO LTD), 15 February 1982 (1982-02-15)**

**Description**

The present invention relates to piping for carrying fluids in a petrochemical cracker between the cracking furnace, through the quench tower, to the cracked gas compressor.

It is already known that fluid can flow in a "swirl flow", and this flow is discussed in WO 97/28637, in the context of penstocks and draft tubes for turbines. The swirl flow is achieved by forming the penstocks or draft tubes in such a way that their centrelines curve in three dimensions.

Swirl flow has a number of advantages over conventional flow. Pressure losses (and energy losses) through turbulence can be reduced. In addition, the velocity profile of the flow across the pipe is more uniform (or blunter) than it would be with conventional flow. As a result, fluid flowing in a swirl flow tends to act as a plunger, removing sediment or debris which may have accumulated on the pipe walls, which is of particular importance in hydroelectric plant.

Pipes having similar three-dimensional curves are also discussed in WO 02/093063, which discloses the preamble of claim 1, where they are used in the context of production and processing plant. In such plant, it is often necessary for pipes connecting various parts of the plant to extend for some distance, and have a number of bends. Forming the bends so that they have three-dimensional curves promotes swirl flow, and leads to reduced energy losses, reduced risk of stagnation and of sedimentation.

However, these prior art documents are only concerned with using three-dimensional curves in place of the known two-dimensional curves (such as elbow bends), so as to induce swirl flow. They are not concerned with creating swirl flow in situations where a generally straight pipe would normally be used.

One possible way of making flow swirl in a straight pipe would be to form grooves or ribs along the inner surface of the pipe, which grooves or ribs curve along the pipe (much like rifling in a gun barrel). However, this has the disadvantage of increasing the wetted perimeter of the pipe, and in the case of ribs, reducing the cross-sectional area of the pipe; both grooves and ribs can lead to increased flow resistance and consequent pressure loss.

In addition, experiment has shown that unless the Reynolds number is very low, the grooves or ribs only have an effect on the flow near the wall of the pipe, and it may be necessary to provide a long pipe in order to be sure that the flow swirls across the entire width of the pipe. Swirl in the centre of the pipe is only achieved through diffusional transfer of momentum from the flow at the wall of the pipe; the grooves or ribs do not facilitate mixing between fluid near the wall of the pipe and fluid at the centre of the pipe.

It is known from US-A-5 167 483 to provide piping comprising a portion wherein the centreline of the portion follows a substantially helical path. GB-A-2 192 966 discloses a method of making piping comprising a portion wherein the centreline of the portion follows a substantially helical path, the method including the steps of providing a shaping apparatus and shaping the pipe into a helical form using the shaping apparatus.

According to a first aspect of the invention, there is provided a petrochemical cracker as defined by claim 1.

When fluid enters a piece of piping shaped as a helical portion in this way, swirl flow is established almost immediately. It has been found that swirl flow is established across the entire width of the pipe within a few pipe diameters of the entry. Further, the swirl flow involves considerable secondary motion and mixing of the fluid, with mass, momentum and heat transfer between the fluid at the walls of the pipe and the fluid at the centre of the pipe.

In this specification, the amplitude of the helix refers to the extent of displacement from a mean position to a lateral extreme. So, in the case of tubing having a helical centre line, the amplitude is one half of the full lateral width of the helical centre line. The cross-sectional area of the tubing is substantially constant along its length.

In the petrochemical cracker with piping according to the first aspect of the invention, there is a "line of sight" along the lumen of the piping. This is distinct from a corkscrew configuration, where the helix is effectively wound around a core (either solid, or "virtual" with a core of air). It has been found that the flow at the line of sight generally has a swirl component, even though it could potentially follow a straight path.

For the purposes of this specification, the term "relative amplitude" of helical piping is defined as the amplitude divided by the internal diameter. Since the amplitude of the helical piping is less than or equal to one half of the internal diameter of the tubing, this means that the relative amplitude is less than or equal to 0.5. Relative amplitudes less than or equal to 0.45, 0.40, 0.35, 0.30, 0.25, 0.20, 0.15, 0.1 or 0.05 may be preferred. Smaller relative amplitudes provide a better use of available lateral space, in that the piping is not much wider overall than a normal straight pipe with the same cross-sectional area. Smaller relative amplitudes also result in a wider "line of sight", providing more space for the insertion of pressure gauges or other equipment along the piping. With higher Reynolds numbers, smaller relative amplitudes may be used whilst swirl flow is induced to a satisfactory extent. This will generally mean that, for a given internal diameter, where there is a high flow rate a low relative amplitude can be used whilst still being sufficient to induce swirl flow.

The angle of the helix is also a relevant factor in balancing space considerations with the desirability of having a large cross-sectional area available for flow. The helix angle is preferably less than or equal to 65°, more preferably less than or equal to 55°, 45°, 35°, 25°, 20°, 15°, 10° or 5°. As with relative amplitudes, the helix angle may be optimized according to the conditions, and in particular the viscosity, density and velocity of the fluid being carried by the piping.

Generally speaking, for higher Reynolds numbers the helix angle may be smaller whilst satisfactory swirl flow is achieved, whilst with lower Reynolds numbers a higher helix angle will be required to produce satisfactory swirl. The use of higher helix angles for faster flows (with higher Reynolds numbers) will generally be undesirable, as there may be near wall pockets of stagnant fluid. Therefore, for a given Reynolds number (or range of Reynolds numbers), the helix angle will preferably be chosen to be as low as possible to produce satisfactory swirl. In certain embodiments, the helix angle is less than 20°.

In general, the piping will have a plurality of turns of the helix. Repeated turns of the helix along the piping will tend to ensure that the swirl flow is fully developed.

Lengths of piping will normally be made with substantially the same relative amplitude and helix angle along their length; however, one or both of them may vary. Further, the helical portion may extend along the entire length of the piping, or may only extend along part of it, to "condition" the flow and to simplify connection of the piping to other pipes.

The piping may extend generally linearly (ie the axis of helical rotation may be a straight line). However, the axis may be curved, to produce a generally curved pipe. The curve of the axis may be two-dimensional or three-dimensional; if it is three-dimensional, then it is important to ensure that the swirl created by the three-dimensional curve augments the swirl created by the helical piping.

According to a further aspect of the invention, there is provided a method of making piping and using the piping in a petrochemical cracker as defined by claim 13.

This method has the advantage of directly producing a helical portion from raw material, and avoids the need to shape a previously formed straight pipe. It can also produce continuous lengths of helical pipe.

In a preferred form, the shaping apparatus comprises a rotating member, whose axis of rotation is generally parallel to the axis of extrusion, which rotating member has a hole therein through which the pipe passes, the hole being positioned so that its centre is offset from the axis of rotation, the rotating member being driven to rotate as the pipe passes through it to impart a helical shape to the pipe, and wherein

the hole in the rotating member is positioned so that the axis of rotation passes through the hole, so as to produce a helical portion wherein the amplitude of the helix is less than or equal to one half of the internal diameter of the piping and is relatively constant along the length of the portion.

Using this shaping apparatus allows the geometry of the pipe to be varied in several ways. For example, the speed of the extruder can be increased or reduced, as can the rotational speed of the rotating member. Further, different rotating members, with the hole in differing positions, can be used.

Preferred embodiments of the invention will now be described by way of example only and with reference to the accompanying drawings, in which:

Figure 1 is a view of tubing used in experiments on the flow in a helical portion;
Figure 2 is a view similar to that of Figure 1 but concerning a different experiment;
Figure 3 illustrates a method of manufacture of a helical pipe;
Figures 4a to 4e illustrate another method of manufacture of a helical pipe; and
Figures 5a to 5c illustrate another method of manufacture of a helical pipe.

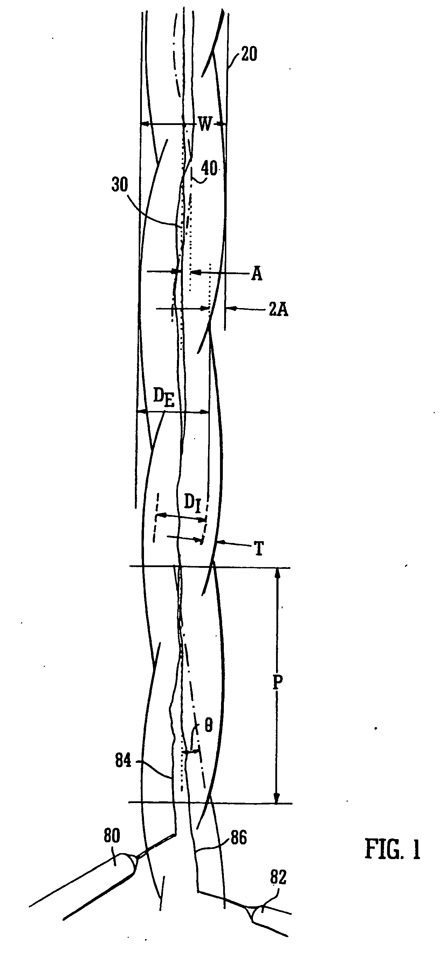

The tubing 10 shown in Figure 1 has a circular cross-section, an external diameter $D_E$, an internal diameter $D_I$ and a wall thickness T. The tubing is coiled into a helix of constant amplitude A (as measured from mean to extreme), constant pitch P, constant helix angle θ and a swept width W. The tubing 10 is contained in an imaginary envelope 20 which extends longitudinally and has a width equal to the swept width W of the helix.

The envelope 20 may be regarded as having a central longitudinal axis 30, which may also be referred to as an axis of helical rotation. The illustrated tubing 10 has a straight axis 30, but it will be appreciated that this axis may instead have a large radius of curvature (either in two or three dimensions). The tubing has a centre line 40 which follows a helical path about the central longitudinal axis 30.

It will be seen that the amplitude A is less than half the tubing internal diameter $D_I$. By keeping the amplitude below this size, the lateral space occupied by the tubing and the overall length of the tubing can be kept relatively small, whilst at the same time the helical configuration of the tubing promotes swirl flow of fluid along the tubing.

A number of experiments were carried out using polyvinyl chloride tubing with a circular cross-section, to establish the characteristics of the flow in a helical portion.

## EXAMPLE 1

Referring to the parameters shown in Figure 1 the tubing had an external diameter $D_E$ of 12mm, an internal diameter $D_I$ of 8mm and a wall thickness T of 2mm. The tubing was coiled into a helix with a pitch P of 45mm and a helix angle θ of 8°. The amplitude A was established by resting the tubing between two straight edges and measuring the space between the straight edges. The amplitude was determined by subtracting the external diameter $D_E$ from the

swept width W:

$$2A = W - D_E$$

So:

$$A = \frac{W - D_E}{2}$$

In this example the swept width W was 14 mm, so:

$$A = \frac{W - D_E}{2} = \frac{14 - 12}{2} = 1 \ mm$$

As discussed earlier, "relative amplitude" $A_R$ is defined as:

$$A_R = \frac{A}{D_I}$$

In the case of this Example, therefore:

$$A_R = \frac{A}{D_I} = \frac{1}{8} = 0.125$$

Water was passed along the tube. In order to observe the flow characteristics, two needles 80 and 82 passing radially through the tube wall were used to inject visible dye into the flow. The injection sites were near to the central axis 30, i.e. at the "core" of the flow. One needle 80 injected red ink and the other needle 82 blue ink. It will be seen in Figure 1 that the ink filaments 84 and 86 intertwine, indicating that in the core there is swirl flow, i.e. flow which is generally helical. The experiment shown in Figure 1 was carried out at a Reynolds number $R_E$ of 500. In two further experiments, respectively using Reynolds numbers of 250 and 100, swirling core flow was also observed.

## EXAMPLE 2

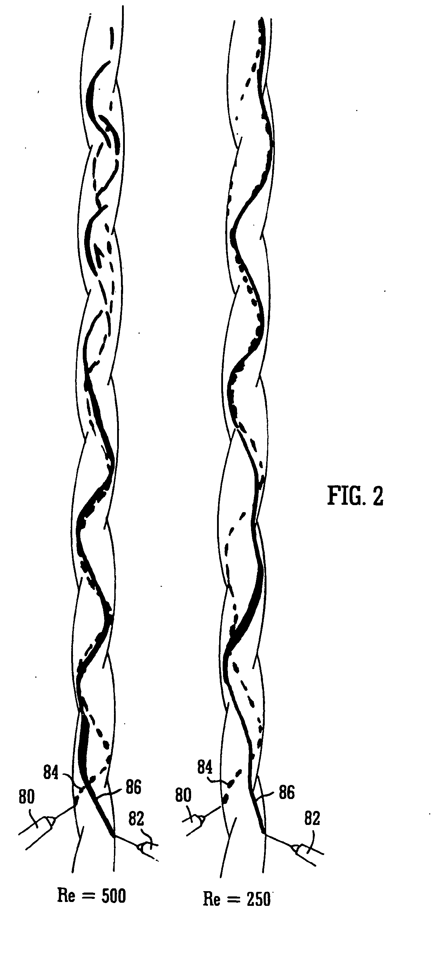

The parameters for this Example were the same as in Example 1, except that the needles 80 and 82 were arranged to release the ink filaments 84 and 86 near to the wall of the tubing. Figure 2 shows the results of two experiments with near-wall ink release, with Reynolds numbers $R_E$ of 500 and 250 respectively. It will be seen that in both cases the ink filaments follow the helical tubing geometry, indicating near-wall swirl.

## EXAMPLE 3

In a separate study, the flow was compared in a straight 8 mm internal diameter tube with that in a helical 8 mm internal diameter tube, where the relative amplitude $A_R$ was 0.45. In both cases the Reynolds number was 500 and 0.2 ml indicator was injected as a bolus through a thin tube at the upstream end. The flows were photographed together with a digital clock to indicate elapsed time after the injection of indicator.

The bolus of indicator, injected into the helical portion, had limited axial dispersion along the pipe, tending to remain coherent. In contrast, in a straight pipe, indicator in the core fluid (near the centre of the pipe) exited the pipe quickly, whereas indicator in fluid near to the walls tended to remain at the walls of the pipe, and took a longer time to exit the pipe. Moreover, the indicator travelled in a more compact mass in the helical tube than in the straight tube. All these findings imply that there was mixing over the tube cross section and blunting of the velocity profile in the helical tube.

## EXAMPLE 4

The experiments of this Example involved a comparison of multi-phase flows in helical tubing with that in tubing having a centreline following a generally sinusoidal path in a single plane. In the case of the helical tubing (whose centre-line curved in three dimensions, i.e. 3D tubing), the internal diameter was 8 mm, the external diameter was 12 mm and the swept width was 17 mm, giving a relative amplitude of 0.3125. The pitch was 90 mm. In the case of the planar, wave-shaped tubing (whose centre-line curved in two dimensions, i.e. 2D tubing), the internal diameter was 8 mm, the external diameter was 12 mm, and the swept width, measured in the plane of the wave shape, was 17 mm. The pitch was 80 mm, not being significantly different from that of the 3D tubing case. The 2D tubing was held with its generally sinusoidal centreline in a vertical plane, in effect creating upwardly convex and concave U-bends.

Both the 3D and 2D tubes were about 400 mm in length, giving 4 to 5 pitches in each case. With both tubes, studies were performed with water flows of 450 and 900 ml per minute (Reynolds numbers of 1200 and 2400 respectively). A needle was used to introduce in all cases a flow of air at a rate of 3 ml per minute, i.e. 0.66% of the water flow in the 450 ml per minute case and 0.33% in the 900 ml per minute case. The air came from a compressed air line and was injected into the tubes just upstream of the start of the respective 3D and 2D geometries.

In the case of the experiment with the 3D tubing at Reynolds number 1200, the air bubbles were about 2 to 3 mm in size and passed along the tube rapidly. At Reynolds number 2400, the bubbles were larger, about 5 to 7 mm but kept moving along the tube with no tendency to stick.

In the case of the 2D tubing at Reynolds numbers of 1200 and 2400, the bubbles were large, about 3 to 5 mm, and tended to stick in the upwardly convex curves (as viewed from outside the tubing).

The experiment shows that in a multi-phase flow the less dense fluid is carried along the 3D tubing, whereas in equivalent 2D tubing the less dense fluid tends to accumulate in the higher parts of the tubing.

As discussed above, when fluid enters a piece of piping shaped as a helical portion in this way, swirl flow is established very quickly. Further, the swirl flow involves considerable secondary motion and mixing of the fluid, with mass transfer between the fluid at the walls of the pipe and the fluid at the centre of the pipe.

This rapid establishment of swirl flow in the helical portion can be used to "condition" the flow, to provide beneficial effects downstream of the helical portion.

As mentioned above, using a pipe having a three-dimensional curve can be better than using a normal (two-dimensional) elbow bend, as the swirl flow established by the three-dimensional curve provides certain benefits. However, it is not normally possible to simply replace an elbow bend by a pipe having a three-dimensional curve; the inlet and outlet pipes of an elbow bend are normally in the same plane, which is not the case with a pipe having a three-dimensional curve. Thus, if a pipe having a three-dimensional curve is to be used in place of an elbow bend, considerable modification can be required to reposition the inlet and/or outlet pipe.

However, the benefits of swirl flow can be achieved with far less modification if a helical portion as described above is fitted upstream of a normal elbow bend. Swirl flow is established rapidly in the helical portion, and this swirl flow continues in the elbow bend.

Since the helical portion has a low amplitude, it can be used in most places where a straight pipe would be used, to "condition" flow in this way to provide the benefits of swirl flow. It should be noted that its use is not limited to elbow bends; it can also be used before T- or Y-junctions, valves, and indeed any form of pipe fitting.

Conditioning the flow in this way is particularly useful before a blind end. Such blind ends can occur at T- or Y-junctions where one of the branches of the junction is closed off (for example, by a valve). With normal flow, the fluid in the part of the branch before the closure tends to stagnate, which can lead to problems with corrosion and the like. However, if the flow is made to swirl before the junction, the swirl extends into the blind end. This prevents stagnation, and avoids the above problems.

A further way of using the helical portions to condition flow is to use them as repeaters. In certain situations, it may not be necessary to provide a continuous length of helical pipe; instead, a straight pipe may have a number of short helical portions arranged along its length. Each portion will induce swirl flow in the fluid passing through it; however, this swirl flow will tend to die away as the fluid passes along the straight pipe. Providing a number of "repeaters" allows the swirl flow to be re-established, with its concomitant benefits.

Helical pipe portions of this type can be made in a number of ways. For example, a straight flexible tube can be wrapped around a straight rigid member (such as a pole), to form it into a helix. The tube can then be removed from the straight rigid member and stretched along the axis of the helix. This stretching has the effect of "flattening out" the helix, in that the pitch is increased and the amplitude is decreased. However, this "flattening out" can distort the helix, and so this method is not preferred.

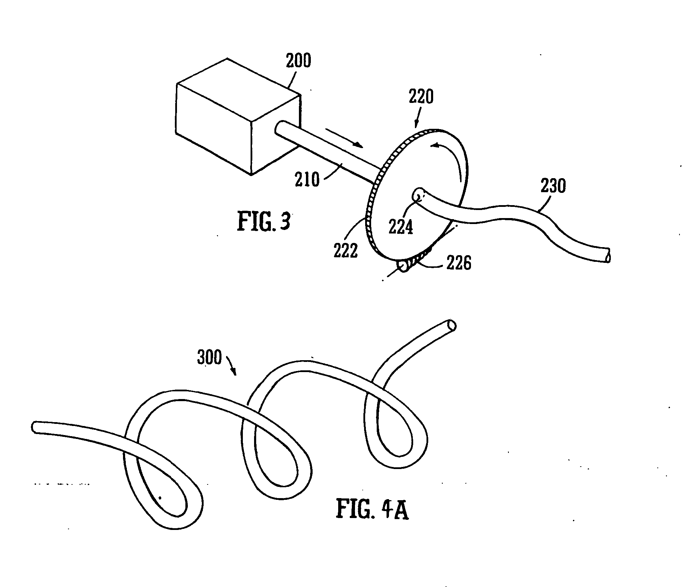

As an alternative to deforming a straight pipe to produce a helical portion, it is possible to form the helical portion directly during extrusion of the pipe. An apparatus for doing this is shown schematically in Figure 3.

As can be seen, the apparatus includes a conventional pipe extruder 200 which extrudes straight pipes 210. Such extruders are well known, and will not be described further.

Disposed downstream of the outlet of the extruder is an apparatus 220 comprising a rotary member 222, which has a through-hole 224. The through-hole is positioned eccentrically, such that the centre of rotation of the rotary member lies within the through-hole, but does not coincide with the centre of the through-hole. The rotary member is held so that the axis of the through-hole is parallel to the axis of the pipe being extruded, and is driven to rotate. This can be achieved by, for example, teeth on the outer periphery of the rotary member which engage with a worm gear 226, or by any other suitable drive system.

The pipe 210 extruded from the extruder is led through the through-hole 224, and as the pipe is extruded, the rotary member 222 is driven to rotate. As a result of this rotation, the centre of the through-hole is driven to describe a circular path, which in turn forces the pipe being extruded into a helical shape. As the through-hole overlies the centre of rotation of the rotary member, the pipe is formed into a small-amplitude helix 230, as described above.

Once the pipe is shaped into the helix, it can be treated to retain its shape. In practice, the pipe can simply be extruded from a thermoplastic material, and as it cools it will set into the helix shape. This cooling may be achieved using water sprays or similar.

It may be necessary to provide some form of lubrication to ensure that the thermoplastic pipe does not stick in the through-hole. In particular, lubrication may be required to ensure that the pipe does not undergo torsion as it passes through the rotary member.

The particular shape of the helix achieved will depend on several factors, in particular the speed of extrusion, the rate of rotation of the rotary member, and the eccentricity of the through-hole. These can be varied to obtain a particular desired form of helical pipe.

A particularly preferred method of forming a helical portion involves the use of a helical mandrel, and is illustrated in Figures 4a to 4e.

Figure 4a is a schematic illustration of a helical mandrel for use in this method. The mandrel consists of a rigid rod, shaped into a helix. In the embodiment shown, the pitch and the amplitude of the helix are constant along the length of the mandrel, but they may vary.

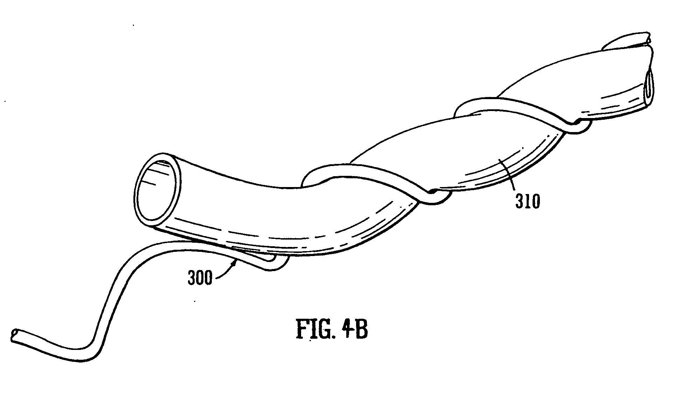

In order to form a helical portion, a length of straight flexible pipe 310, whose external diameter is greater than the internal diameter of the mandrel 300, is wound around the mandrel 300, as shown in Figure 4b. Because the pipe is wider than the space inside the mandrel, it is forced to adopt a helical form, as can be seen from the Figure.

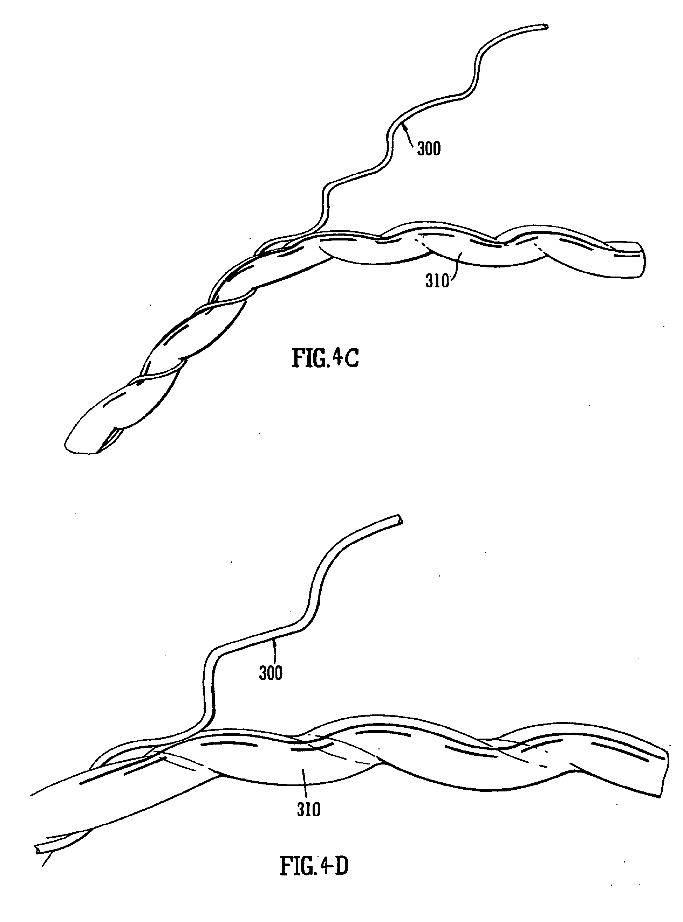

After being treated so that it will retain its helical shape, the pipe can be removed from the mandrel, as shown in Figures 4c and 4d.

As can be seen, the pitch of the helical portion is the same as the pitch of the mandrel. The amplitude of the helical portion will be determined by the diameters of the pipe and of the mandrel.

The above description concerns a batch processing method for forming the helical portion; but this method also lends itself to continuous operation. A continuous length of flexible pipe can be drawn through a comparatively short length of mandrel, and can be treated to retain its shape as it is drawn through (for example, by heating a pipe formed from a thermosetting resin).

Experiment has shown that the pipe rotates relative to the mandrel when it is drawn through in this way. Thus, some form of lubrication may be required to enable smooth functioning of the process. For extremely large pipes and mandrels, it may be desirable to provide roller bearings on the mandrel, rather than lubrication.

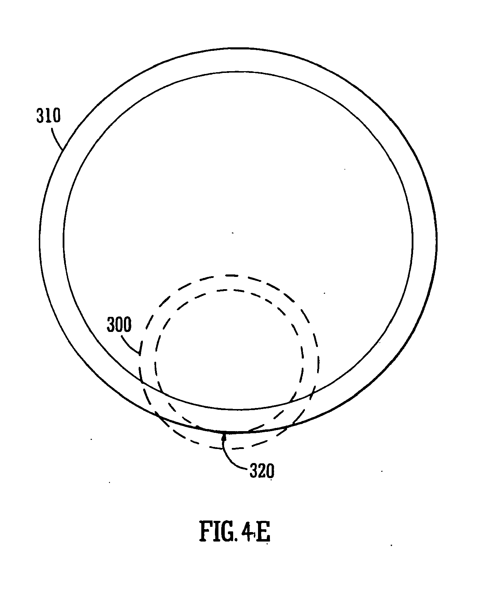

Figure 4e is a schematic cross-section through the pipe 310 and the mandrel 300 as the pipe is drawn. As the helical mandrel is viewed end-on along its axis, it appears as a circle; similarly, the pipe (having a circular cross-section) also appears as a circle in the Figure. It will be seen that the mandrel contacts the outside of the pipe, at point 320, and so the mandrel can be supported from below without interfering with the drawing process.

The mandrel can be formed in any suitable manner, and the method of forming the mandrel will depend to a large extent on the size of the pipes being treated. For relatively small pipes, the mandrel could be formed by winding a rod around a member with a circular cross-section. For larger pipes, the mandrel may need to be machined, for example using a CNC milling machine.

The methods described above are limited to certain materials (such as thermosetting and thermoplastic materials). However, these materials tend to be rather low in strength, and will probably not be suitable for use in more extreme environments, such as offshore, or where very high-pressure fluids must be carried. If a small-amplitude helical pipe is to be used in such situations, then it must be formed in a different manner.

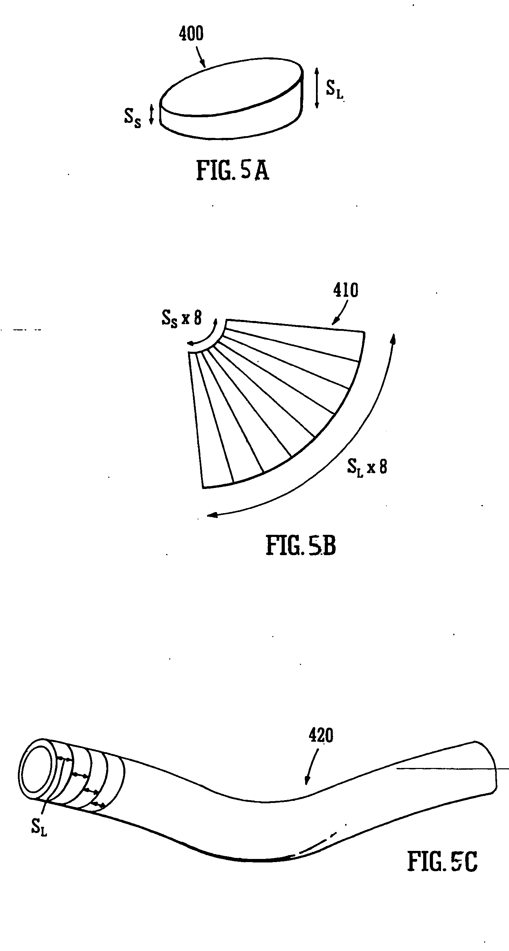

One way of forming a small-amplitude helix for use in high-pressure situations is illustrated with reference to Figures 5a, 5b and 5c.

A known method of forming straight high-pressure pipes is to form them from a large number of short sections, each of which is effectively a very short pipe. Each section has a flange on its upstream and downstream ends, and these flanges co-operate with each other to hold the sections together. In the prior art, the ends of the sections lie in parallel planes, and so when the sections are connected together, the resulting pipe is straight.

However, the segments can also be formed so that their ends lie in planes that are slightly skew. A segment 400 of this type will have one side ($S_L$) which is slightly longer than the diametrically opposite side ($S_S$), as shown in Figure 5a, and can be assembled to form curved pipes, and helical pipes as described above.

To produce a pipe 410 with a two-dimensional curve from short skew-ended pipe sections, the sections are connected so that the longer side of one section connects with the longer side of the previous section, with the shorter sides likewise connecting to each other. As shown in Figure 5b, this produces a pipe with a two-dimensional curve.

To produce a helical pipe 420, the sections are connected together in a similar manner, but each section is slightly rotated relative to the previous section. This is shown in Figure 5c, which shows a helical pipe formed from such sections. At the left-hand of the pipe, the longer sides $S_L$ are shown for the first few sections, and it will be seen that there is relative rotation between the sections. The amount of relative rotation determines the pitch of the helix, with a small relative rotation producing a helix with a small helix angle and a large pitch, and a large relative rotation producing a helix with a large helix angle and a small pitch.

It will be appreciated that at least one end of the pipe will be somewhat elliptical, rather than perfectly circular (as the end is formed by the intersection of a plane cutting a cylinder at an angle to the axis of the cylinder which is not exactly 90o). In a preferred form, both ends are formed so that they are elliptical, as this makes the formation of a two-dimensional curve easier (as the elliptical faces on either end of the segments can match up with each other).

In order to allow the sections to be assembled into a helix, it is necessary for there to be some degree of compliance in the end faces, so that they can accommodate a slight rotation and/or change in shape between the end faces being connected to each other. This can be achieved in any suitable manner, for example by means of an elastomeric material in the end faces.

The effects produced by swirl flow in the helical portion, and in particular the more uniform velocity profile and the improved mixing, can be taken advantage of in a number of situations. In addition, as the overall width of the helical portion is only slightly larger than that of a straight pipe of the same cross-sectional area, the helical portion can be used in virtually any situation where a straight pipe would normally be used.

Further, because of the more uniform velocity profile, and the improved mixing between fluid at the wall of the pipe and fluid at the centre of the pipe, the residence time of fluid in the pipe is much more uniform. This is of considerable advantage if the fluid in the pipe is being treated in some way (for example, heated, cooled, irradiated and so on), as the effects of the treatment on the fluid will be more uniform. By way of contrast, in a normal pipe where flow in the centre of the pipe is faster than flow at the walls of the pipe, the residence time will vary (depending on whether the fluid finds itself near the centre or near the wall). Thus, the fluid near the walls will be treated to a greater degree than the fluid in the centre of the pipe, because of its larger residence time. This can be seen from the discussion of Example 3 above.

Another advantage of the secondary motion and mixing associated with swirling flow in a helix is inhibition of the development of flow instability and turbulence; this has been shown experimentally.

The helical piping portions are used in petrochemical crackers. Many cracking processes produce more molecules than are present in the feedstock, and yields rely on a low pressure environment to prevent the molecules from recombining. This is achieved by cooling products in a quench tower, and minimizing pressure loss between the cracking furnace, through the quench tower, to the cracked gas compressor (as yield is inversely proportional to pressure loss). The use of the helical portions in place of straight pipes can reduce the pressure loss, and thus increase yield.

## Claims

1. A petrochemical cracker comprising a cracking furnace, a quench tower, a cracked gas compressor and piping, the piping comprising a helical piping portion positioned between the cracking furnace, through the quench tower, to the cracked gas compressor, **characterized in that** the centreline (40) of the helical piping portion follows a substantially helical path, the amplitude (A) of the helix being less than or equal to one half of the internal diameter ($D_I$) of the helical piping portion so as to provide a line of sight along the lumen of the piping portion.

2. A petrochemical cracker as claimed in claim 1, **characterized in that** the piping portion has a plurality of turns of the helix.

3. A petrochemical cracker as claimed in claim 1 or claim 2, **characterized in that** the piping portion has a varying amplitude and/or a varying helix angle along its length.

**4.** A petrochemical cracker as claimed in claim 1 or claim 2, **characterized in that** the piping portion has substantially the same amplitude and helix angle along its length.

**5.** A petrochemical cracker as claimed in any preceding claim, **characterized in that** the piping portion extends along the entire length of the piping.

**6.** A petrochemical cracker as claimed in any of claims 1 to 4, **characterized in that** the piping portion only extends along part of the piping.

**7.** A petrochemical cracker as claimed in any preceding claim, **characterized in that** the axis (30) of helical rotation is a straight line.

**8.** A petrochemical cracker as claimed in any of claims 1 to 6, **characterized in that** the axis of helical rotation is curved.

**9.** A petrochemical cracker as claimed in any preceding claim, **characterized in that** the piping portion has a substantially circular cross-section.

**10.** A petrochemical cracker as claimed in any preceding claim, **characterized in that** the amplitude of the helix is less than or equal to 0.4 of the internal diameter ($D_I$) of the piping portion.

**11.** A petrochemical cracker as claimed in any preceding claim **characterized in that** the angle of the helix is less than or equal to 15°.

**12.** A petrochemical cracker as claimed in any preceding claim, **characterized in that** the piping portion has an inner surface with no grooves or ribs.

**13.** A method of making piping, and using the piping in a petrochemical cracker comprising a cracking furnace, a quench tower and a cracked gas compressor, the piping being positioned between the cracking furnace, through the quench tower, to the cracked gas compressor, and comprising a portion wherein the centreline (40) of the piping portion follows a substantially helical path and the amplitude (A) of the helical centreline (40) is less than or equal to one half of the internal diameter ($D_I$) of the piping portion, the method of making the piping including the following steps:

> providing an extruder (200) for extruding a straight tube (210);
> providing a shaping apparatus (220) downstream of said extruder for shaping the extruded tube into the helical form such that the amplitude (A) of the helical centreline (40) is less than or equal to one half of the internal diameter ($D_I$) of the piping portion;
> and extruding a straight tube from the extruder and shaping the tube into the helical form (230) using the shaping apparatus.

**14.** A method as claimed in claim 13, **characterized in that** the shaping apparatus (220) comprises a rotating member (222), whose axis of rotation is generally parallel to the axis of extrusion, which rotating member has a hole (224) therein through which the tube passes, wherein the hole (224) in the rotating member (222) is positioned so that the axis of rotation passes through the hole but is offset from the centre of the hole, so as to produce a helical portion wherein the amplitude of the helix is less than or equal to one half of the internal diameter of the piping and is relatively constant along the length of the portion.


**Patentansprüche**

**1.** Petrochemische Krackanlage, umfassend einen Krackofen, einen Löschturm, einen Krackgasverdichter und ein Rohrleitungssystem, wobei das Rohrleitungssystem einen spiralförmigen Rohrleitungsabschnitt umfasst, der zwischen dem Krackofen, durch den Löschturm zum Krackgasverdichter verlaufend positioniert ist, **dadurch gekennzeichnet, dass** die Mittellinie (40) des spiralförmigen Rohrleitungsabschnitts im Wesentlichen spiralförmig verläuft, wobei die Amplitude (A) der Spirale kleiner als die oder gleich der Hälfte des Innendurchmessers ($D_I$) des spiralförmigen Rohrleitungsabschnitts ist, um eine Sichtlinie entlang des Lumens des Rohrleitungsabschnitts zu schaffen.

**2.** Petrochemische Krackanlage nach Anspruch 1, **dadurch gekennzeichnet, dass** der Rohrleitungsabschnitt eine Mehrzahl von Spiralwindungen aufweist.

3. Petrochemische Krackanlage nach Anspruch 1 oder Anspruch 2, **dadurch gekennzeichnet, dass** der Rohrleitungsabschnitt entlang seiner Länge eine variierende Amplitude und/oder einen variierenden Spiralwinkel aufweist.

4. Petrochemische Krackanlage nach Anspruch 1 oder Anspruch 2, **dadurch gekennzeichnet, dass** der Rohrleitungsabschnitt entlang seiner Länge im Wesentlichen die gleiche Amplitude und den gleichen Spiralwinkel aufweist.

5. Petrochemische Krackanlage nach einem der vorangehenden Ansprüche, **dadurch gekennzeichnet, dass** sich der Rohrleitungsabschnitt entlang der gesamten Länge des Rohrleitungssystems erstreckt.

6. Petrochemische Krackanlage nach einem der Ansprüche 1 bis 4, **dadurch gekennzeichnet, dass** sich der Rohrleitungsabschnitt nur entlang eines Teils des Rohrleitungssystems erstreckt.

7. Petrochemische Krackanlage nach einem der vorangehenden Ansprüche, **dadurch gekennzeichnet, dass** die Achse (30) der spiralförmigen Windung eine gerade Linie ist.

8. Petrochemische Krackanlage nach einem der Ansprüche 1 bis 6, **dadurch gekennzeichnet, dass** die Achse der spiralförmigen Windung gekrümmt ist.

9. Petrochemische Krackanlage nach einem der vorangehenden Ansprüche, **dadurch gekennzeichnet, dass** der Rohrleitungsabschnitt einen im Wesentlichen kreisförmigen Querschnitt aufweist.

10. Petrochemische Krackanlage nach einem der vorangehenden Ansprüche, **dadurch gekennzeichnet, dass** die Amplitude der Spirale kleiner als oder gleich 0,4 des Innendurchmessers ($D_I$) des Rohrleitungsabschnitts ist.

11. Petrochemische Krackanlage nach einem der vorangehenden Ansprüche, **dadurch gekennzeichnet, dass** der Spiralwinkel kleiner als oder gleich 15° ist.

12. Petrochemische Krackanlage nach einem der vorangehenden Ansprüche, **dadurch gekennzeichnet, dass** der Rohrleitungsabschnitt eine Innenfläche ohne Nuten oder Rippen aufweist.

13. Verfahren zur Herstellung eines Rohrleitungssystems und Verwendung des Rohrleitungssystems in einer petrochemischen Krackanlage, die einen Krackofen, einen Löschturm und einen Krackgasverdichter umfasst, wobei das Rohrleitungssystem zwischen dem Krackofen, durch den Löschturm zum Krackgasverdichter verlaufend positioniert ist und einen Abschnitt umfasst, in dem die Mittellinie (40) des Rohrleitungsabschnitts im Wesentlichen spiralförmig verläuft und die Amplitude (A) der spiralförmigen Mittellinie (40) kleiner als die oder gleich der Hälfte des Innendurchmessers ($D_I$) des Rohrleitungsabschnitts ist, wobei das Verfahren zur Herstellung des Rohrleitungssystems folgende Schritte einschließt:

   Bereitstellen eines Extruders (200) zum Extrudieren eines geraden Rohrs (210);
   Bereitstellen eines dem Extruder nachgeschalteten Formgebungsgeräts (220) zum Formen des extrudierten Rohrs, so dass es die Spiralform erhält, derart dass die Amplitude (A) der spiralförmigen Mittellinie (40) kleiner als die oder gleich der Hälfte des Innendurchmessers ($D_I$) des Rohrleitungsabschnitts ist;
   und Extrudieren eines geraden Rohrs durch den Extruder und Formen des Rohrs, so dass es die Spiralform (230) erhält, unter Verwendung des Formgebungsgeräts.

14. Verfahren nach Anspruch 13, **dadurch gekennzeichnet, dass** das Formgebungsgerät (220) ein sich drehendes Element (222) umfasst, dessen Drehachse im Allgemeinen parallel zur Extrusionsachse ist, wobei das sich drehende Element mit einem Loch (224) versehen ist, durch welches das Rohr verläuft, wobei das Loch (224) in dem sich drehenden Element (222) so positioniert ist, dass die Drehachse durch das Loch verläuft, aber gegenüber der Mitte des Lochs versetzt ist, um einen spiralförmigen Abschnitt zu erzeugen, in dem die Amplitude der Spirale kleiner als die oder gleich der Hälfte des Innendurchmessers des Rohrleitungssystems und entlang der Länge des Abschnitts relativ konstant ist.

**Revendications**

1. Craqueur pétrochimique comprenant un four de craquage, une tour de trempe, un compresseur de gaz craqué et une canalisation, la canalisation comprenant une portion de canalisation hélicoïdale positionnée entre le four de craquage et le compresseur de gaz craqué en passant par la tour de trempe, **caractérisé en ce que** la ligne médiane (40) de la portion de canalisation hélicoïdale suit un trajet sensiblement hélicoïdal, l'amplitude (A) de l'hélice étant inférieure ou égale à une moitié du diamètre interne ($D_1$) de la portion de canalisation hélicoïdale de manière à fournir une ligne de visée le long de la lumière de la portion de canalisation.

2. Craqueur pétrochimique selon la revendication 1, **caractérisé en ce que** la portion de canalisation a une pluralité de spires de l'hélice.

3. Craqueur pétrochimique selon la revendication 1 ou la revendication 2, **caractérisé en ce que** la portion de canalisation a une amplitude variable et/ou un angle d'hélice variable sur sa longueur.

4. Craqueur pétrochimique selon la revendication 1 ou la revendication 2, **caractérisé en ce que** la portion de canalisation a sensiblement la même amplitude et le même angle d'hélice sur sa longueur.

5. Craqueur pétrochimique selon l'une quelconque des revendications précédentes, **caractérisé en ce que** la portion de canalisation s'étend sur toute la longueur de la canalisation.

6. Craqueur pétrochimique selon l'une quelconque des revendications 1 à 4, **caractérisé en ce que** la portion de canalisation ne s'étend que sur une partie de la canalisation.

7. Craqueur pétrochimique selon l'une quelconque des revendications précédentes, **caractérisé en ce que** l'axe (30) de rotation hélicoïdale est une ligne droite.

8. Craqueur pétrochimique selon l'une quelconque des revendications 1 à 6, **caractérisé en ce que** l'axe de rotation hélicoïdale est incurvé.

9. Craqueur pétrochimique selon l'une quelconque des revendications précédentes, **caractérisé en ce que** la portion de canalisation a une section transversale sensiblement circulaire.

10. Craqueur pétrochimique selon l'une quelconque des revendications précédentes, **caractérisé en ce que** l'amplitude de l'hélice est inférieure ou égale à 0,4 fois le diamètre interne ($D_1$) de la portion de canalisation.

11. Craqueur pétrochimique selon l'une quelconque des revendications précédentes, **caractérisé en ce que** l'angle de l'hélice est inférieur ou égal à 15°.

12. Craqueur pétrochimique selon l'une quelconque des revendications précédentes, **caractérisé en ce que** la portion de canalisation a une surface interne sans rainures ni nervures.

13. Procédé de fabrication de canalisation et utilisation de la canalisation dans un craqueur pétrochimique comprenant un four de craquage, une tour de trempe et un compresseur de gaz craqué, la canalisation étant positionnée entre le four de craquage et le compresseur de gaz craqué en passant par la tour de trempe, et comprenant une portion dans laquelle la ligne médiane (40) de la portion de canalisation suit un trajet sensiblement hélicoïdal et l'amplitude (A) de la ligne médiane hélicoïdale (40) est inférieure ou égale à la moitié du diamètre interne ($D_1$) de la portion de canalisation, le procédé de fabrication de la canalisation comprenant les étapes consistant à :

fournir une extrudeuse (200) pour extruder un tube droit (210) ;
fournir un appareil de mise en forme (220) en aval de ladite extrudeuse pour mettre en forme le tube extrudé afin de lui donner la forme hélicoïdale de sorte que l'amplitude (A) de ligne médiane hélicoïdale (40) soit inférieure ou égale à une moitié du diamètre interne ($D_1$) de la portion de canalisation ; et
extruder un tube droit de l'extrudeuse et mettre en forme le tube pour lui donner la forme hélicoïdale (230) en utilisant l'appareil de mise en forme.

14. Procédé selon la revendication 13, **caractérisé en ce que** l'appareil de mise en forme (220) comprend un organe rotatif (222), dont l'axe de rotation est généralement parallèle à l'axe d'extrusion, lequel organe rotatif présente un trou (224) par lequel le tube passe, dans lequel le trou (224) de l'organe rotatif (222) est positionné de sorte que l'axe de rotation passe à travers le trou, mais soit décalé du centre du trou, de manière à produire une portion hélicoïdale dans laquelle l'amplitude de l'hélice est inférieure ou égale à une moitié du diamètre interne de la canalisation et est relativement constante sur la longueur de la portion.

FIG. 1

FIG. 2

Re = 500          Re = 250

FIG. 3

FIG. 4A

300

310

FIG. 4B

300

310

**FIG.4C**

300

310

**FIG.4D**

310

300

320

FIG. 4E

400

$S_S$ $\downarrow$  $\uparrow$ $S_L$

FIG. 5A

$S_S$ x 8    410

$S_L$ x 8

FIG. 5B

420

$S_L$

FIG. 5C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9728637 A
- WO 02093063 A
- US 5167483 A
- GB 2192966 A